# FASCICULE DE BREVET EUROPEEN

(11) **EP 1 989 321 B1**
(45) Date de publication et mention de la délivrance du brevet: **04.12.2013**
(21) Numéro de dépôt: 07731662.8
(22) Date de dépôt: 23.02.2007
(51) Int. Cl.: C12Q 1/04, C12N 1/20, C12Q 1/37, C12Q 1/42, C12Q 1/44

(54) **PROCÉDÉ POUR L'IDENTIFICATION D'AU MOINS DEUX GROUPES DE MICROORGANISMES**
VERFAHREN ZUR IDENTIFIZIERUNG VON MINDESTENS ZWEI MIKROORGANISMENGRUPPEN
METHOD FOR IDENTIFYING AT LEAST TWO GROUPS OF MICROORGANISMS

(30) Priorité: 28.02.2006 FR 0650693
(43) Date de publication de la demande: 12.11.2008
(60) Demande divisionnaire: 11192535.0
(73) Titulaire: BIOMERIEUX, 69280 Marcy L'Etoile (FR)
(72) Inventeur: JAMES, Arthur, Cumbria CA13 9 UX (GB); ORENGA, Sylvain, F-01160 Neuville Sur Ain (FR); PERRY, John, Newcastle-upon-tyne NE2HR (GB); ROGER-DALBERT, Céline, F-01150 Chazey Sur Ain (FR)
(74) Mandataire: Bitaud, Valérie Marie-Odile
(86) Numéro de dépôt international: PCT/FR2007/050844
(87) Numéro de publication internationale: WO 2007/099254

(56) Documents cités:
- EP-A- 1 382 689
- EP-A1- 0 451 775
- WO-A-01/30794
- WO-A-02/44402
- FR-A1- 2 659 982
- FR-A1- 2 763 342
- US-A- 4 874 695

## Description

L'invention concerne un procédé pour l'identification d'au moins deux groupes de microorganismes exprimant une même activité enzymatique. L'invention concerne également un milieu réactionnel particulier ainsi que son utilisation pour l'identification d'au moins deux groupes de microorganismes exprimant une même activité enzymatique.

Depuis de très nombreuses années, on utilise des substrats enzymatiques particuliers pour permettre la détermination de la présence ou de l'absence d'activités enzymatiques caractéristiques de micro-organismes. Ces substrats enzymatiques, sont généralement composés de deux parties, une première partie spécifique de l'activité enzymatique à révéler, appelée également partie cible, et une seconde partie faisant office de marqueur, appelée partie marqueur, induisant par exemple une coloration particulière de la colonie lors de l'hydrolyse du substrat, ou l'apparition d'un précipité aisément détectable. Par le choix de ces substrats, selon qu'il y a réaction ou non, par exemple une coloration différente, il est possible de caractériser la nature d'un microorganisme, ou de discriminer différents groupes de microorganismes. Ainsi, dans le cas de bactéries, les souches *d'Escherichia coli* sont souvent mises en évidence par la révélation d'une activité enzymatique du type osidase telle que l'activité β-glucuronidase ou β-galactosidase. De la même façon, le genre *Listeria* peut être détecté par la mise en évidence d'une activité β-glucosidase. On peut citer également la détection d'une activité estérase pour notamment la mise en évidence du genre *Salmonella.* En effet, le genre *Salmonella* possède des estérases non spécifiques capables d'hydrolyser des substrats synthétiques chromogènes, par exemple indigogéniques. Dans le cas de la détection de salmonelles, et plus généralement dans le cas de bactéries à activité estérase, la détection et/ou l'identification de ces bactéries est classiquement réalisée sur des milieux gélosés, qui permettent la détection et/ou l'identification des colonies suspectes de bactéries à activité estérase.

Toutefois, une activité enzymatique unique n'est pas toujours suffisante pour caractériser un groupe particulier de microorganismes d'un autre groupe de microorganismes. Par exemple, si l'on veut différencier des bactéries gram positive et des bactéries gram négative, comme par exemple les bactéries du groupe KES (*Klebsiella, Enterobacter* et *Serratia,* bactéries à Gram négatif), et celles du genre *Enterococcus* (bactéries à Gram positif), il est nécessaire de détecter plusieurs activités enzymatiques pour accroître la spécificité. Il convient alors de combiner plusieurs substrats enzymatiques dans un même milieu de culture, ce qui peut engendrer un coût élevé de fabrication. De plus, dans certains cas, il n'est pas possible de révéler spontanément dans un même milieu réactionnel des activités différentes car les conditions d'expression des différentes activités ne sont pas compatibles. Ainsi, lors de l'utilisation du milieu chromogène CPS ID 3 commercialisé par bioMérieux, les bactéries du groupe KES (*Klebsiella, Enterobacter et Serratia,* bactéries à Gram négatif), comme celles du genre *Enterococcus* (bactéries à Gram positif), détectées par une activité osidase (beta glucosidase) produisent des colonies bleu-vert : la distinction entre les deux groupes doit être confirmée par une étape supplémentaire, par un examen microscopique.

Le document US 4 874 695 divulgue une méthode pour l'identification de microorganismes de type champignons et levures. Ladite méthode comprend les étapes suivantes culture des champignons ou levures inconnus; préparation d'un inoculum desdits microorganismes inconnus; mélange, séparément, de l'inoculum avec un ou plusieurs substrats enzymatiques chromogènes.

Le document EP 0 451 775 divulgue une méthode rapide et précise d'identification d'un microorganisme. Ladite méthode comprenant les étapes consistant à: déterminer le taux de clivage enzymatique d'une pluralité de substrats par au moins une enzyme exprimée par un microorganisme non identifié en présence d'une concentration effective d'au moins un effecteur; identifier ledit microorganisme par comparaison du profil de taux de clivage enzymatique avec les profils équivalents de microorganismes connus.

Le document FR 2 659 982 divulgue un procédé d'identification de deux levures caractérisé en ce qu'il met en oeuvre au moins un substrat chromogène sensible à au moins une enzyme et un système de moyens inhibiteurs (caractéristique essentielle). FR 2 659 982 reste muet sur la mise en oeuvre de deux substrats différents pour une même activité enzymatique

L'invention se propose de résoudre les problèmes de l'état de la technique en présentant un nouveau procédé particulièrement adapté pour identifier, discriminer spécifiquement différents groupes de microorganismes d'une manière rapide, peu coûteuse et aisée à mettre en oeuvre.

De façon surprenante, les inventeurs ont mis en évidence qu'il était possible de différencier deux groupes de micro-organismes exprimant une même activité enzymatique, par le choix judicieux d'une combinaison de substrats spécifiques de l'activité enzymatique exprimée par les deux groupes de micro-organismes.

Avant d'aller plus avant dans l'exposé de l'invention, les définitions suivantes sont données afin de faciliter la compréhension de l'invention :
Au sens de la présente invention, le terme microorganisme recouvre les bactéries,
gram positif ou gram négatif, les levures et plus généralement, les organismes généralement unicellulaires, invisibles à l'oeil nu, qui peuvent être multipliés et manipulés en laboratoire.

A titre de bactéries à Gram négatif, on peut citer les bactéries des genres suivants : *Pseudomonas, Escherichia, Salmonella, Shigella, Enterobacter, Klebsiella, Serratia, Proteus, Campylobacter, Haemophilus, Morganella, Vabrio, Yersinia, Acinetobacter, Branhamella, Neisseria, Burkholderia, Citrobacter, Hafnia, Edwardsiella, Aeromonas, Moraxella, Pasteurella. Providencia Actinobacillus Alcaligenes, Bordetella, Cedecea, Erwinia, Pantoea, Ralstonia, Stenotrophomonas, Xanthomonas* et *Legionella.*

A titre de bactéries à Gram positif, on peut citer les bactéries des genres suivants : *Enterococcus, Streptococcus, Staphylococcus, Bacillus, Listeria, Clostridium, Gardnerella, Kocuria, Lactococcus, Leuconostoc, Micrococcus, Mycobacteria* et *Corynebacteria*

A titre de levures, on peut citer les levures des genres suivants : *Candida, Cryptococcus, Rhodotorula, Saccharomyces* et *Trichosporon.*

Par milieu réactionnel, on entend un milieu comprenant tous les éléments nécessaires à l'expression d'un métabolisme et/ou à la croissance de microorganismes. Ce milieu réactionnel peut soit servir uniquement de milieu de révélation, soit de milieu de culture et de révélation. Dans le premier cas, la culture des microorganismes est effectuée avant ensemencement et, dans le deuxième cas, le milieu réactionnel constitue également le milieu de culture. Ce milieu peut contenir d'éventuels autres additifs comme par exemple : des peptones, un ou plusieurs facteurs de croissance, des hydrates de carbone, un ou plusieurs agents sélectifs, des tampons, un ou plusieurs gélifiants... Ce milieu réactionnel peut se présenter sous forme de liquide, de gel prêt à l'emploi, c'est à dire prêt à l'ensemencement en tube, flacon, ou sur boite de Petri.

Par substrat enzymatique, on entend tout substrat pouvant être hydrolysé par une enzyme en un produit permettant la détection, directe ou indirecte d'un microorganismes. Ce substrat comprend notamment une première partie spécifique de l'activité enzymatique à révéler et une seconde partie faisant office de marqueur, ci-après appelée partie marqueur. Cette partie marqueur peut être chromogène, fluorogène, luminescente... Comme substrat chromogène, bien adapté aux supports solides (filtre, gélose, gel d'électrophorèse), on peut citer notamment les substrats à base d'Indoxyl et ses dérivés, et les substrats à base d'Hydroxyquinoline ou d'Esculétine et leurs dérivés, qui permettent la détection d'activités osidase et estérase.

A titre de substrats à base d'Indoxyl, on peut citer notamment : 5-Bromo-4-chloro-3-indolyl-N-acétyl-ß-D-glucosamine, 5-Bromo-3-indolyl-N-acétyl-ß-D-glucosamine, 6-Chloro-3-indolyl-N-acétyl-ß-D-glucosamine, 5-Bromo-6-chloro-3-indolyl-N-acétyl-ß-D-glucosamine, 5-Bromo-4-chloro-3-indolyl-N-acétyl-ß-D-galacto-samine, 5-Bromo-4-chloro-3-indolyl-ß-D-cellobioside, 5-Bromo-3-indolyl-ß-D-cellobioside, 6-Chloro-3-indolyl-ß-D-cellobioside, 5-Bromo-6-chloro-3-indolyl-ß-D-cellobioside, 5-Bromo-4-chloro-3-indolyl-ß-D-galactoside, 5-Bromo-3-indolyl-ß-D-galactoside, 6-Chloro-3-indolyl-ß-D-galactoside, 5-Bromo-6-chloro-3-indolyl-ß-D-galactoside, 6-Bromo-3-indolyl-ß-D-galactoside, 3-Indoxyl-ß-D-galactoside, 5-Bromo-4-chloro-3-indolyl-α-D-galactoside, 5-Bromo-3-indolyl-α-D-galactoside, 6-Chloro-3-indolyl-α-D-galactoside, 5-Bromo-6-chloro-3-indolyl-α-D-galactoside, 5-Bromo-4-chloro-3-indolyl-ß-D-glucoside, 5-Bromo-3-indolyl-ß-D-glucoside, 6-Chloro-3-indolyl-ß-D-glucoside, 5-Bromo-6-chloro-3-indolyl-ß-D-glucoside, 5-Bromo-4-chloro-3-indolyl-N-méthyl-ß-D-glucoside, 6-Bromo-3-indolyl-6-D-glucoside, 3-Indoxyl-ß-D-glucoside, 5-Bromo-4-chloro-3-indolyl-α-D-glucoside, 5-Bromo-3-indolyl-α-D-glucoside, 6-Chloro-3-indolyl-α-D-glucoside, 5-Bromo-6-chloro-3-indolyl-α-D-glucoside, 5-Bromo-4-chloro-3-indolyl-N-méthyl-α-D-glucoside, 5-Bromo-4-chloro-3-indolyl-ß-D-glucuronide, 5-Bromo-3-indolyl-ß-D-glucuronide, 6-Chloro-3-indolyl-ß-D-glucuronide, 5-Bromo-6-chloro-3-indolyl-ß-D-glucuronide, 6-Bromo-3-indolyl-ß-D-glucuronide, 3-Indoxyl-ß-D-glucuronide, 5-Bromo-4-chloro-3-indolyl-α-D-mannoside, 5-Bromo-6-chloro-3-indolyl-α-D-mannoside, 6-Chloro-3-indolyl-α-D-mannoside, 5-Bromo-4-chloro-3-indolyl-ß-D-mannoside, 5-Bromo-6-chloro-3-indolyl-ß-D-mannoside, 5-Bromo-4-chloro-3-indolyl-0-D-riboside, 5-Bromo-4-chloro-3-indolyl-ß-L-fucoside, 5-Bromo-4-chloro-3-indolyl-ß-D-xyloside, 5-Bromo-6-chloro-3-indolyl-ß-D-xyloside, 5-Bromo-4-chloro-3-indolyl-myo-inositol-1-phosphate, 5-Bromo-4-chloro-3-indoxyl-phosphate, 5-Bromo-3-indoxyl-ß-D-phosphate, 6-Chloro-3-indoxyl-phosphate, 5-Bromo-6-chloro-3-indoxyl-phosphate, 3-Indoxyl-phosphate, 5-Bromo-4-chloro-3-indoxyl-acétate, 5-Bromo-3-indoxyl-ß-D-acétate, 6-Chloro-3-indoxyl-acétate, 5-Bromo-6-chloro-3-indoxyl-acétate, 5-Bromo-4-chloro-3-indoxyl-butyrate, 5-Bromo-3-indoxyl-ß-D-butyrate, 6-Chloro-3-indoxyl-butyrate, 5-Bromo-6-chloro-3-indoxyl-butyrate, 5-Bromo-4-chloro-3-indoxyl-octanoate, 5-Bromo-3-indoxyl-ß-D-octanoate, 6-Chloro-3-indoxyl-octanoate, 5-Bromo-6-chloro-3-indoxyl-octanoate, 5-Bromo-4-chloro-3-indoxyl-nonanoate, 5-Bromo-3-indoxyl-ß-D-nonanoate, 6-Chloro-3-indoxyl-nonanoate, 5-Bromo-6-chloro-3-indoxyl-nonanoate, 5-Bromo-4-chloro-3-indoxyl-décanoate, 5-Bromo-3-indoxyl-ß-D-décanoate, 6-Chloro-3-indoxyl-décanoate, 5-Bromo-4-chloro-3-indoxyl-oléate, 5-Bromo-4-chloro-3-indoxyl-palmitate, 5-Bromo-4-chloro-3-indoxyl-sulfate, 5-Bromo-6-chloro-3-indoxyl-sulfate.

On peut également citer les substrats dérivés de flavoides. Par dérivé de flavoide, on entend notamment le 3',4'-Dihydroxyflavone-4'-ß-D-riboside, le 3',4'-Dihydroxyflavone-4'-ß-D-galactoside, le 3',4'-Dihydroxyflavone-4'-ß-D-glucoside, 3-Hydroxyflavone-ß-D-galactoside, le 3-Hydroxyflavone-ß-D-glucoside, 3',4'-Dihydroxyflavone-3',4'-diacétate.

On peut citer également les substrats à base de Nitrophénol et Nitroaniline et dérivés, permettant de détecter les activités osidases et estérases dans le cas de substrats à base de Nitrophénol, et des activités peptidases dans le cas de substrats à base de la Nitroaniline. On peut aussi citer les substrats à base de Coumarine et dérivés permettant aussi de détecter les activités osidases et estérases dans le cas de substrats à base d'hydroxycoumarines et notamment de la 4-Méthyl-umbelliférone ou de la Cyclohexenoesculétine, et des activités peptidases dans le cas de substrats à base d'aminocoumarines et notamment de la 7-Amino-4-méthyl-coumarine. On peut encore citer les substrats à base d'Aminophénol et dérivés permettant de détecter les activités osidases, estérases et peptidases. On peut citer également les substrats à base d'Alizarine et dérivés permettant de détecter les activités osidases et estérases. On peut citer enfin les substrats à base de Naphtol et Naphtylamine et leurs dérivés, qui permettent de détecter les activités osidases et estérases par l'intermédiaire du Naphtol, et les activités peptidases par l'intermédiaire de la Naphtylamine

Par substrat à base de Naphtol, on entend notamment les substrats à bases d'α-Naphtol, de ß-Naphtol, de 6-Bromo-2-naphtol, de Naphtol AS BI, de Naphtol AS, de p-Naphtolbenzeine tel que définis dans la demande de brevet EP1224196 de la demanderesse. Cela peut être des substrats d'osidase, d'estérase, de phosphatase, de sulfatase. Les substrats d'osidase sont notamment des substrats de N-Acétyl-ß-hexosaminidase, de ß-galactosidase, d'α-galacotosidase, de ß-glucosidase, d'α-glucosidase, de ß-glucucronidase, de ß-cellobiosidase, d'α-mannosidase.

Par substrat à base d'Alizarine, on entend notamment les substrats décrits dans le brevet EP1235928 de la demanderesse, c'est à dire un substrat de formule générale : 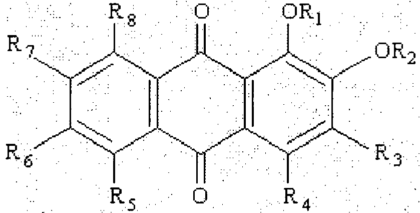 dans laquelle :
- R₁ est une partie cible ou H et R₂ est une partie cible ou H, au moins l'un des R₁ et R₂ étant une partie cible,
- R₃ est H, SO₃H, Cl, Br, F, I, NO₂, NH₂, NR₉R₁₀, Acylamino, Aminoaryl ou Aminoacylamino du type NHCOX, avec X égal à Alkyl, Aryl et Aralkyl ou un résidu d'acide α-aminé, tel que l'Alanine,
- R₄ est H, SO₃H, Cl, Br, F, I, NO₂, NH₂, NR₉R₁₀, OH, Acylamino, Aminoaryl ou Aminoacylamino du type NHCOX, avec X égal à Alkyl, Aryl, Aralkyl ou un résidu d'acide α-aminé, tel que l'Alanine,
- selon une variante, R₃ et R₄ sont reliés l'un à l'autre pour former un cycle à au moins cinq côtés, et préférentiellement à six côtés,
- R₅, R₆, R₇ et R₈ sont chacun constitués d'un des atomes ou groupements d'atomes suivants : H, halogène, particulièrement Cl ou Br, OH, SO₃H, Alkyl ou Alkoxy, et
- R₉ et R₁₀ sont constitués indépendamment de Méthyl, Alkyl, Aryl, Aralkyl ou constituent l'un, R₉ ou R₁₀, un cycle (Pipéridine, Pyrrolidine, Morpholine, etc.) et l'autre, R₁₀ ou R₉, un atome d'Hydrogène..

Le substrat enzymatique peut également être un substrat naturel dont le produit d'hydrolyse est détecté directement ou indirectement. Comme substrat naturel, on peut notamment citer le Tryptophane pour détecter une activité tryptophanase ou desaminase, un acide aminé cyclique (Tryptophane, Phénylalanine, Histidine, Tyrosine) pour détecter une activité désaminase, le Phosphatidyl Inositol pour détecter une activité phospholipase, ...

A cet effet, la présente invention concerne un procédé pour l'identification d'au moins deux groupes de microorganismes exprimant une même activité enzymatique comprenant les étapes suivantes :
a) l'incubation desdits groupes de microorganisme dans un milieu réactionnel comprenant un premier substrat enzymatique et un deuxième substrat enzymatique, lesdits premier et deuxième substrats enzymatiques étant métabolisés par une même activité enzymatique
b) l'identification desdits groupes de microorganismes

D'une manière générale, les étapes d'incubation et d'identification sont largement connues de l'homme du métier. Par exemple, la température d'incubation peut être de 37°C. S'agissant de l'atmosphère d'incubation, elle est préférentiellement aérobie, mais elle peut également être anaérobie, microaérobie ou sous CO₂. L'identification peut être mise en oeuvre à l'oeil nu par visualisation d'un changement de coloration, ne diffusant pas dans le milieu réactionnel, donc concentrée au niveau des colonies. Dans le cas de la révélation de la fluorescence, on utilise les dispositifs de lecture de la fluorescence connus de l'homme du métier.

Préférentiellement, ladite même activité enzymatique est choisie parmi les activités enzymatiques suivantes : osidase, estérase, peptidase, et encore plus préférentiellement, ladite même activité enzymatique est choisie parmi les activités enzymatiques suivantes : ß-D-glucosidase, ß-D-galactosidase, alpha-D-glucosidase, alpha-D-galactosidase, alpha-mannosidase, ß-D-glucuronidase, N-Acetyl-ß-D-hexosaminidase, ß-D-cellobiosidase, estérase, phosphatase, phospholipase, sulfatase, peptidase.

Ledit premier groupe de microorganisme est un groupe de *Staphylococcus aureus* et ledit deuxième groupe est un groupe de *Enterococcus faecalis,* ladite même activité enzymatique est un activité alpha glucosidase, et lesdits premier et deuxième substrats sont à base d'Indoxyl.

Le premier substrat est le 5-Bromo-4-chloro-3-indolyl-N-méthyl-alpha-glucoside et le deuxième substrat est le 6-Chloro-3-indolyl-alpha-glucoside Lesdits premier et deuxième groupes de microorganismes sont des salmonelles de sérotypes différents, ladite même activité enzymatique est une activité estérase, lesdits premier et deuxième substrats sont à base d'Indoxyl.

Ledit premier substrat est le 5-Bromo-4-chloro-3-indoxyl-octanoate et ledit deuxième substrat est le 5-Bromo-6-chloro-3-indoxyl-octanoate Selon un autre mode préféré de réalisation de l'invention, ledit premier groupe de microorganismes est un groupe de bactéries gram + et ledit deuxième groupe de microorganismes est un groupe de bactéries gram - ; ladite même activité enzymatique est une activité beta glucosidase ; ledit premier substrat est un dérivé de flavoide et ledit deuxième substrat est à base d'Indoxyl.

Ledit premier substrat est le 3-Hydroxyflavone-beta-glucoside, et ledit deuxième substrat est le 5-Bromo-4-chloro-N-methyl-3-indolyl-beta-glucoside. Selon un autre mode préféré de réalisation de l'invention, ledit premier groupe de microorganismes est un groupe de bactéries gram + et ledit deuxième groupe de microorganismes est un groupe de bactéries gram - ; ladite même activité enzymatique est une activité beta glucuronidase ; ledit premier substrat est à base de Naphtol et ledit deuxième substrat est à base d'Indoxyl.

Ledit premier substrat est le p-Naphtholbenzeïne-beta-glucuronide et ledit deuxième substrat est le 6-Chloro-3-indolyl-beta-glucuronide.

Selon un autre mode préféré de réalisation de l'invention, ledit premier groupe de microorganismes est un groupe de levures et ledit deuxième groupe de microorganismes est un groupe de bactéries ; ladite même activité enzymatique est une activité hexosaminidase ; ledit premier substrat est à base d'Alizarine et ledit deuxième substrat est à base d'Indoxyl.

Préférentiellement, ledit premier substrat est l'Alizarine-N-acétyl-beta glucosaminide et ledit deuxième substrat est le 5-Bromo-4-chloro-3-indolyl-N-acetyl-beta-glucosaminide.

Quelque soit le mode de réalisation du procédé selon l'invention, le milieu réactionnel peut comprendre, en outre, au moins un autre substrat, préférentiellement plusieurs, métabolisé par au moins une autre activité enzymatique, préférentiellement plusieurs, ladite autre activité enzymatique étant préférentiellement choisi parmi une activité β-D-glucuronidase, une activité β-glucosidase, une activité tryptophanase, une activité désaminase. Selon un mode encore plus préféré, ledit autre substrat est choisi parmi le 6-Chloro-3-indolyl-beta-glucuronide, le 5-Bromo-4-chloro-3-indolyl-N-méthyl-β-D glucoside, le 3',4'-Dihydroxy-4'-β-D-glucoside, et le tryptophane.

Il est ainsi possible de discriminer et d'identifier non seulement un premier groupe de bactéries gram - et un deuxième groupe de bactéries gram + exprimant toutes une activité β-glucosidase, mais il est également possible d'identifier au moins un troisième groupe de bactéries exprimant une activité β-glucuronidase et un quatrième groupe de bactéries exprimant une activité désaminase, ainsi que des sous-groupes exprimant une activité tryptophanase.

La demande concerne également l'utilisation d'un milieu réactionnel comprenant au moins un premier substrat enzymatique et au moins un deuxième substrat enzymatique, lesdits premier et deuxième substrat enzymatiques étant métabolisés par une même activité enzymatique, pour l'identification d'au moins deux groupes de microorganismes, préférentiellement un premier groupe de microorganismes et un deuxième groupe de microorganismes, exprimant une même activité enzymatique.

Préférentiellement, l'invention concerne l'utilisation d'un milieu réactionnel comprenant au moins un premier substrat enzymatique et au moins un deuxième substrat enzymatique, lesdits premier et deuxième substrats enzymatiques étant métabolisés par une même activité enzymatique, pour l'identification d'un premier groupe de microorganismes et d'un deuxième groupe de microorganismes, exprimant une même activité enzymatique.

Préférentiellement, ladite même activité enzymatique est choisie parmi les activités enzymatiques suivantes : osidase, estérase, peptidase, et encore plus préférentiellement, ladite même activité enzymatique est choisie parmi les activités enzymatiques suivantes : ß-D-glucosidase, ß-D-galactosidase, alpha-D-glucosidase, alpha-D-galactosidase, alpha-mannosidase, ß-D-glucuronidase, N-Acetyl-ß-D-hexosaminidase, ß-D-cellobiosidase, estérase, phosphatase, phospholipase, sulfatase, peptidase.

Ledit premier groupe de microorganisme est un groupe de *S*. *aureus* et ledit deuxième groupe est un groupe de *E. faecalis,* ladite même activité enzymatique est un activité alpha glucosidase, et lesdits premier et deuxième substrats sont à base d'Indoxyl. Le premier substrat est le 5-Bromo-4-chloro-3-indolyl-N-méthyl-alpha-glucoside et le deuxième substrat est le 6-Chloro-3-indolyl-alpha-glucoside. Selon un autre mode préféré de réalisation de l'invention, lesdits premier et deuxième groupes de microorganismes sont des salmonelles de sérotypes différents, ladite même activité enzymatique est une activité estérase, lesdits premier et deuxième substrats sont à base d'Indoxyl.

Ledit premier substrat est le 5-Bromo-4-chloro-3-indoxyl-octanoate et ledit deuxième substrat est le 5-Bromo-6-chloro-3-indoxyl-octanoate. Selon un autre mode préféré de réalisation de l'invention, ledit premier groupe de microorganismes est un groupe de bactéries gram + et ledit deuxième groupe de microorganismes est un groupe de bactéries gram - ; ladite même activité enzymatique est une activité beta glucosidase ; ledit premier substrat est un dérivé de flavoide et ledit deuxième substrat est à base d'Indoxyl.

Préférentiellement, ledit premier substrat est le 3-Hydroxyflavone-beta-glucoside, et ledit deuxième substrat est le 5-Bromo-4-chloro-N-methyl-3-indolyl-beta-glucoside. Selon un autre mode préféré de réalisation de l'invention, ledit premier groupe de microorganismes est un groupe de bactéries gram + et ledit deuxième groupe de microorganismes est un groupe de bactéries gram - ; ladite même activité enzymatique est une activité beta glucuronidase ; ledit premier substrat est à base de Naphtol et ledit deuxième substrat est à base d'Indoxyl.

Ledit premier substrat est le p-Naphtholbenzeïne-beta-glucuronide et ledit deuxième substrat est le 6-Chloro-3-indolyl-beta-glucuronide Selon un autre mode préféré de réalisation de l'invention, ledit premier groupe de microorganismes est un groupe de levures et ledit deuxième groupe de microorganismes est un groupe de bactéries ; ladite même activité enzymatique est une activité hexosaminidase ; ledit premier substrat est à base d'Alizarine et ledit deuxième substrat est à base d'Indoxyl.

Ledit premier substrat est l'Alizarine-N-acétyl-beta-glucosaminide et ledit deuxième substrat est le 5-Bromo-4-chloro-3-indolyl N-acetyl-beta-glucosaminide.

Le milieu réactionnel peut comprendre, en outre, au moins un autre substrat, préférentiellement plusieurs, métabolisé par au moins une autre activité enzymatique, préférentiellement plusieurs, ladite autre activité enzymatique étant préférentiellement choisi parmi une activité β-D-glucuronidase, une activité β-glucosidase, une activité tryptophanase, une activité désaminase. Selon un mode encore plus préféré, ledit autre substrat est choisi parmi le 6-Chloro-3-indolyl-beta-glucuronide, le 5-bromo-4-chloro-3-indolyl-N-méthyl-β-D-glucoside, le 3',4'-Dihydroxy-4'-β-D-glucoside, et le tryptophane.

Il est ainsi possible de discriminer et d'identifier non seulement un premier groupe de bactéries gram - et un deuxième groupe de bactéries gram + exprimant toutes une activité ß-glucosidase, mais il est également possible d'identifier au moins un troisième groupe de bactéries exprimant une activité ß-glucuronidase et un quatrième groupe de bactéries exprimant une activité désaminase, ainsi que des sous-groupes exprimant une activité tryptophanase. Un tel milieu est très utile, notamment pour le diagnostic des infections urinaires.

La demande concerne également un milieu réactionnel comprenant au moins un premier substrat enzymatique et au moins un deuxième substrat enzymatique, lesdits premier et deuxième substrats enzymatiques étant métabolisés par une même activité enzymatique.

Lesdits premier et deuxième substrats sont à base d'Indoxyl, préférentiellement le premier substrat est le 5-Bromo-4-chloro-3-indolyl-N-méthyl-alpha-glucoside et le deuxième substrat est le 6-Chloro-3-indolyl-alpha-glucoside. Un tel milieu est particulièrement adapté pour identifier un groupe de *S*. *aureus* et un groupe de *E. faecalis.*

Selon un autre mode préféré de réalisation de l'invention, lesdits premier et deuxième substrats sont à base d'Indoxyl, préférentiellement ledit premier substrat est le 5-Bromo-4-chloro-3-indoxyl-octanoate et ledit deuxième substrat est le 5-Bromo-6-chloro-3-indoxyl-octanoate. Un tel milieu est particulièrement adapté pour identifier deux groupes de salmonelles de sérotype différents.

Ledit premier substrat est un dérivé de flavoide, préférentiellement le 3-Hydroxyflavone-beta-glucoside et ledit deuxième substrat est à base d'Indoxyl, préférentiellement le 5-Bromo-4-chloro-N-methyl-3-indolyl-beta-glucoside. Un tel milieu est particulièrement adapté pour identifier un groupe de bactéries gram + d'un groupe de bactéries gram -.

Ledit premier substrat est à base de Naphtol et ledit deuxième substrat est à base d'Indoxyl, préférentiellement ledit premier substrat est le p-Naphtholbenzeïne-beta-glucuronide et ledit deuxième substrat est le 6-Chloro-3-indolyl-beta-glucuronide. Un tel milieu est particulièrement adapté pour identifier un groupe de bactéries gram + d'un groupe de bactéries gram -.

Ledit premier substrat est à base d'Alizarine et ledit deuxième substrat est à base d'Indoxyl, préférentiellement, ledit premier substrat est l'Alizarine-N-acétyl-beta-glucosaminide et ledit deuxième substrat est le 5-Bromo-4-chloro-3-indolyl N-acetyl-beta-glucosaminide. Un tel milieu est particulièrement adapté pour identifier un groupe de bactéries d'un groupe de levures.

Le milieu réactionnel peut comprendre, en outre, au moins un autre substrat, préférentiellement plusieurs, métabolisé par au moins une autre activité enzymatique, préférentiellement plusieurs, ladite autre activité enzymatique étant préférentiellement choisi parmi une activité β-D-glucuronidase, une activité β-glucosidase, une activité tryptophanase, une activité désaminase. Selon un mode encore plus préféré, ledit autre substrat est choisi parmi le 6-Chloro-3-indolyl-beta-glucuronide, le 5-Bromo-4-chloro-3-indolyl-N-méthyl-β-D-glucoside, le 3',4'-Dihydroxy-4'-β-D-glucoside, et le tryptophane.

Il est ainsi possible de discriminer et d'identifier non seulement un premier groupe de bactéries gram - et un deuxième groupe de bactéries gram + exprimant toutes une activité ß-glucosidase, mais il est également possible d'identifier au moins un troisième groupe de bactéries exprimant une activité ß-glucuronidase et un quatrième groupe de bactéries exprimant une activité désaminase, ainsi que des sous-groupes exprimant une activité tryptophanase.

Les exemples suivants sont donnés à titre illustratif et n'ont aucun caractère limitatif. Ils permettront de mieux comprendre l'invention.

### Exemple 1 : Test pour déterminer un différentiel de métabolisme d'un même substrat par différents groupes de microorganismes exprimant une même activité enzymatique

L'objectif est de différencier au moins deux groupes de micro-organismes, par exemple un premier groupe et un deuxième groupe, exprimant une même activité enzymatique donnée, par exemple une activité enzymatique d'une enzyme alpha. L'activité de chacun des deux groupes de micro-organismes est évaluée vis à vis de différents substrats de l'enzyme alpha, par exemple un substrat A, un substrat B, un substrat C. Chacun des substrats A, B, C est ajouté individuellement à un milieu réactionnel adapté au métabolisme dudit premier groupe et dudit deuxième groupe de micro-organismes que l'on souhaite différencier. On obtient ainsi un milieu A, comprenant le substrat A, un milieu B comprenant le substrat B, un milieu C comprenant le substrat C...

A titre indicatif, pour discriminer des levures, un milieu adapté au métabolisme des levures peut être notamment un milieu Sabouraud éventuellement dépourvu partiellement ou totalement de glucose.

Un milieu adapté au métabolisme des bactéries peut être notamment un milieu Trypcase Soja ou un milieu Columbia.

Un milieu adapté au métabolisme des micro-organismes urinaires, peut être notamment un milieu CPS ID 3, dépourvu de ses substrats enzymatiques.

Bien entendu, en fonction des micro-organismes à différencier et de l'activité enzymatique étudiée, d'autres milieux réactionnels pourront être privilégiés. Suivant l'application, il pourra s'agir de milieux liquides ou de milieux gélifiés.

Chaque milieu est aliquoté. Une ou plusieurs souches de chacun des groupes de micro-organismes à différencier est ensemencée sur une aliquote de chaque milieu. Ces cultures sont ensuite incubées dans les conditions appropriées.

L'hydrolyse de chacun des substrats est évaluée, éventuellement après différentes durées d'incubation, de façon à déterminer s'il existe entre au moins deux substrats d'une même activité enzymatique, un différentiel d'hydrolyse lié au groupe de micro-organismes.

Lorsqu'un tel différentiel est identifié, un milieu réactionnel analogue additionné des substrats enzymatiques présentant ce différentiel d'hydrolyse est fabriqué. Comme précédemment, il est aliquoté. Une ou plusieurs souches de chacun des groupes de micro-organismes à différencier est ensemencée sur une aliquote de ce milieu. Ces cultures sont ensuite incubées dans les conditions appropriées puis examinées, éventuellement après différentes durée d'incubation, pour évaluer si le différentiel d'expression enzymatique permet de différencier les groupes de micro-organismes étudiés. En effet la différenciation des groupes dépend non seulement du différentiel d'hydrolyse entre les substrats, mais également de la différence entre les signaux produits par l'hydrolyse de chacun des substrats et d'éventuelles interactions notamment au niveau des substrats enzymatiques et/ou des signaux produits. L'exemple développé précédemment peut être mis en ouvre d'une manière similaire pour discriminer non pas deux groupes de microorganismes, mais 3, 4 ou plus, groupes de microorganismes.

### Exemple 2 : Utilisation de deux substrats d'alpha glucosidase à base d'Indoxyl le 5-Bromo-4-chloro-3-indolyl-N-méthyl-alpha-glucoside (X-N-Me-α-GLU) en combinaison avec le 6-Chloro-3-indolyl-alpha-glucoside (Rose-α-GLU) pour discriminer les espèces Staphylococcus aureus et Enterococcus faecalis

Un volume de 200 ml d'agar Columbia fondu à 50°C a été ajouté à différentes compositions en substrats décrites dans le tableau 1 ci-dessous :

**Tableau 1**

| | Milieu 1 | Milieu 2 | Milieu 3 |
|---|---|---|---|
| X-N-Me-α-GLU | 75 mg/l | - | 75 mg/l |
| Rose-α-GLU | - | 200 mg/l | 200 mg/l |

Les milieux ainsi constitués ont été répartis dans des boîtes de Petri.

Trois souches différentes de *S*. *aureus* et d'*E. faecalis* ont été ensemencées, à l'öese calibrée de 10 µl, sur chaque milieu, à partir de suspensions calibrées de 0,5 McFarland. Toutes les cultures ont été incubées 24 h à 37°C.

Les résultats de croissance et de coloration obtenus à 24 h d'incubation sont donnés dans le tableau 2, dans lequel Co signifie croissance, C signifie couleur, I signifie incolore, In signifie intensité de coloration, le sigle ++ signifie très bonne croissance, le sigle + signifie bonne croissance de la souche, le sigle +/- signifie croissance moyenne de la souche et le sigle - signifie absence de croissance de la souche. 1 correspond à une intensité de coloration faible, 2 moyenne 3 forte.

**Tableau 2**

| | | Milieu 1 | | | Milieu 2 | | | Milieu 3 | | |
|---|---|---|---|---|---|---|---|---|---|---|
| | | C | In | Co | C | In | Co | C | In | Co |
| 1 | *S. aureus* | Vert | 1 | + | Rose | 2 | + | Violet | 3 | + |
| 2 | *S. aureus* | Vert | 1 | + | Rose | 2 | + | Violet | 1 | + |
| 3 | *S. aureus* | Vert | 2 | + | Rose | 2 | + | Violet | 2 | + |
| 4 | *E. faecium* | I | | + | I | | + | I | | + |
| 5 | *E. faecalis* | Vert | 3 | + | Rose | 1 | + | Vert | 3 | + |
| 6 | *E. faecalis* | Vert | 3 | + | rose | 1 | + | vert | 2 | + |

Les résultats dans le tableau 2 ci-dessus montrent que les deux espèces testées *S. aureus* et *E. faecalis* présentent des intensités de coloration différentes pour chaque substrat La combinaison dans un même milieu de ces deux substrats permet d'observer des colorations différentes pour chaque espèce Ainsi pour une même activité enzymatique et en présence de deux substrats de cette activité, il est possible de différencier deux espèces pour lesquelles l'affinité de leur enzyme à chaque substrat est variable De plus, si on ajoute un glycopeptide, par exemple de la vancomycine ou de la teicoplanine à une concentration adaptée, par exemple entre 2 et 16mg/l, ce milieu est particulièrement adapté à la détection des cocci Gram positif résistants aux glycopeptides, notamment ceux ayant une résistante dite acquise, tel que *Staphylococcus aureus* résistant à la Vancomycine (VRSA) ou *Enterococcus faecalis* ou *E. faecium* résistant à la Vancomycine (VRE).

### Exemple 3 : Utilisation de deux substrats d'estérase à base d'Indoxyl le 5-Bromo-4-chloro-3-indoxyl-octanoate (X-C8) en combinaison avec le 5-Bromo-6-chloro-3-indoxyl-octanoate (Magenta-C8) pour discriminer des souches de salmonelles de sérotypes différents

Un volume de 200 ml d'agar Columbia fondu à 50°C a été ajouté à différentes compositions en substrats résumées dans le tableau 3 ci-dessous :

**Tableau 3**

| | Milieu 1 | Milieu 2 | Milieu 3 |
|---|---|---|---|
| X-C8 | 300 mg/l | - | 300 mg/l |
| Magenta-C8 | - | 500 mg/l | 500 mg/l |

Les milieux ainsi constitués ont été répartis dans des boîtes de Petri

Différents sérotypes de salmonelles ont été ensemencés à l'öese calibrée de 10 µl, sur chaque milieu, à partir de suspensions calibrées de 0,5 McFarland. Toutes les cultures ont été incubées 24 h à 37°C

Les résultats de croissance et de coloration obtenus à 24 h d'incubation sont donnés dans le tableau 3, dans lequel Co signifie croissance, C signifie couleur, I signifie incolore, In signifie intensité de coloration, le sigle ++ signifie très bonne croissance, le sigle + signifie bonne croissance de la souche, le sigle +/- signifie croissance moyenne de la souche et le sigle - signifie absence de croissance de la souche 1 correspond à une intensité de coloration faible, 2 moyenne 3 forte.

**Tableau 4**

| | | Milieu 1 | | | Milieu 2 | | | Milieu 3 | | |
|---|---|---|---|---|---|---|---|---|---|---|
| | | C | In | Co | C | In | Co | C | In | Co |
| 1 | *S. enteritidis* | Vert | 1 | + | I | | + | Vert | 1 | + |
| | sérot. Dublin | | | | | | | | | |
| 2 | *S. enteritidis* | Vert | 1 | + | I | | + | Vert | 1 | + |
| | sérot. Dublin | | | | | | | | | |
| 3 | *S. enteritidis* | Vert | 2 | + | Violet | 2 | + | Violet | 2 | + |
| | sérot. Enteritidis | | | | | | | | | |
| 4 | *S. enteritidis* | vert | 3 | + | Violet | 2 | + | violet | 3 | + |
| | sérot Enteritidis | | | | | | | | | |
| 5 | *S. enteritidis* | Vert | 1 | + | violet | 1 | + | Violet | 1 | + |
| | sérot. Paratyphi A | | | | | | | | | |
| 6 | *S. enteritidis* | vert | 1 | + | Violet | 3 | + | Violet | 3 | + |
| | sérot. Typhimurium | | | | | | | | | |

Les résultats dans le tableau 4 montrent que l'affinité de l'activité estérase des différents sérotypes de salmonelles est variable selon les substrats Le sérotype Dublin hydrolyse plus spécifiquement le X-C8 que le Magenta-C8 A l'inverse, les autres sérotypes hydrolysent préférentiellement le Magenta-C8, les intensités de coloration sont plus fortes pour ce substrat. De ce fait un milieu contenant ces deux substrats d'une même activité enzymatique permet d'obtenir des colonies colorées pour les différents sérotypes de salmonelles, de séparer le sérotype Dublin des autres sérotypes de salmonelles et de bactéries n'exprimant pas d'estérase

### Exemple 4: Utilisation d'un substrat de beta glucosidase 3-Hydroxyflavone-beta-glucoside (HF-β-GLU) ou de 3',4'-Di-hydroxyflavone-beta-glucoside (DHF-β-GLU) avec un substrat à base d'Indoxyl le 5-Bromo-4-chloro-N-methyl-3-indolyl-beta-glucoside (X-N-Me-β-GLU) pour discriminer des bactéries gram + et des bactéries gram - possédant une activité beta-glucosidase Un volume de 200 ml d'agar Columbia fondu à 50°C a été ajouté à différentes compositions en substrats résumées dans le tableau 5

**Tableau 5**

| | Milieu 1 | Milieu 2 | Milieu 3 |
|---|---|---|---|
| X-N-Me-β-GLU | 75 mg/l | 75 mg/l | 75 mg/l |
| HF-β-GLU | - | - | 200 mg/l |
| DHF-β-GLU | - | 200 mg/l | - |

Les milieux ainsi constitués ont été répartis dans des boîtes de Petri. Des souches de *Enterobacter cloacae, Klebsiella pneumoniae*, *Serratia marcescens, Enterococcus faecium*, *E. faecalis*, *Enterococcus gallinarum*, *S. aureus*, *E. coli*, ont été ensemencées, à l'öese calibrée de 10 µl, sur chaque milieu, à partir de suspensions calibrées de 0,5 McFarland. Toutes les cultures ont été incubées 24 h à 37°C

Les résultats de croissance et de coloration obtenus à 24 h d'incubation sont donnés dans le tableau 4, dans lequel Co signifie croissance, C signifie couleur, I signifie incolore, In signifie intensité de coloration, le sigle ++ signifie très bonne croissance, le sigle + signifie bonne croissance de la souche, le sigle +/- signifie croissance moyenne de la souche et le sigle - signifie absence de croissance de la souche. 1 correspond à une intensité de coloration faible, 2 moyenne, 3 forte et 4 très forte.

**Tableau 6**

| | | Milieu 1 | | | Milieu 2 | | | Milieu 3 | | |
|---|---|---|---|---|---|---|---|---|---|---|
| | | C | In | Co | C | In | Co | C | In | Co |
| 1 | *E. cloacae* | Vert | 3 | + | Vert | 3 | + | Vert | 3 | + |
| 2 | *K. pneumoniae* | Vert | 3 | + | Vert | 3 | + | Vert | 3 | + |
| 3 | *S*. *marcescens* | Vert | 2 | + | Vert | 2 | + | Vert | 2 | + |
| 4 | *E. faecium* | vert | 4 | + | Noir | 4 | + | Violet avec reflets métalliques | 4 | + |
| 5 | *E. faecalis* | Vert | 4 | + | Noir | 4 | + | Violet avec reflets métalliques | 4 | + |
| 6 | *E. gallinarum* | Vert | 4 | + | Noir | 4 | | Violet avec reflets métallique | 4 | |
| 7 | *S. aureus* | 1 | | + | I | | + | I | | + |
| 8 | *E. coli* | I | | + | I | | + | I | | + |

Les résultats dans le tableau 6 montrent que les espèces à Gram négatif et beta-glucosidase positive hydrolysent préférentiellement le substrat à base d'indoxyl alors que les espèces à Gram positif et beta-glucosidase positive hydrolysent préférentiellement les substrats à base de flavoïdes. De ce fait il est possible de séparer des bactéries à Gram positif et à Gram négatif présentant la même activité enzymatique. En effet la différenciation des groupes dépend du différentiel d'hydrolyse entre les substrats, mais également de la différence entre les signaux produits par l'hydrolyse de chacun des substrats.

Exemple 5 : Utilisation d'un substrat de p-Naphtholbenzeïne-beta-glucuronide (pNB-β-GUR) avec un substrat à base d'indoxyl le 6-Chloro-3-indolyl-beta-glucuronide (Rose-β-GUR) pour discriminer des bactéries gram + et des bactéries gram - possédant une activité beta-glucuronidase

Un volume de 200 ml d'agar Columbia fondu à 50°C a été ajouté à différentes compositions en substrats résumées dans le tableau 7 ci-dessous.

**Tableau 7**

| | Milieu 1 | Milieu 2 | Milieu 3 |
|---|---|---|---|
| pNB-β-GUR | | 50 mg/l | 50 mg/l |
| Rose-β-GUR | 200 mg/l | - | 200 mg/l |

Les milieux ainsi constitués ont été répartis dans des boîtes de Petri.

Différentes souches de microorganismes ont été ensemencées, à l'öese calibrée de 10 µl, sur chaque milieu, à partir de suspensions calibrées de 0,5 McFarland. Toutes les cultures ont été incubées 24 h à 37°C.

Les résultats de croissance et de coloration obtenus à 24 h d'incubation sont donnés dans le tableau 5, dans lequel Co signifie croissance, C signifie couleur, I signifie incolore, In signifie intensité de coloration, le sigle ++ signifie très bonne croissance, le sigle + signifie bonne croissance de la souche, le sigle +/- signifie croissance moyenne de la souche et le sigle - signifie absence de croissance de la souche. 1 correspond à une intensité de coloration faible, 2 moyenne, 3 forte et 4 très forte.

**Tableau 8**

| | | Milieu 1 | | | Milieu 2 | | | Milieu 3 | | |
|---|---|---|---|---|---|---|---|---|---|---|
| | | C | In | Co | C | In | Co | C | In | Co |
| 1 | *E. coli* | Rose | 1 | + | I | | + | Rose | 1 | + |
| 2 | *E. coli* | Rose | 3 | + | I | | + | Rose | 3 | + |
| 3 | *Streptococcus agalactiae* | Rose | 1 | + | orange | 1 | + | orange | 1 | + |
| 4 | *Streptococcus agalactiae* | Rose | 2 | + | orange | 2 | + | orange | 2 | + |
| 5 | *S. enteritidis* | I | | + | I | | + | I | | + |

Les résultats dans le tableau 8 montrent que les espèces à Gram négatif et beta-glucuromdase positive hydrolysent préférentiellement le substrat à base d'indoxyl alors que les espèces à Gram positif et beta-glucuronidase positive hydrolysent préférentiellement les substrats à base de p-Naphtolbenzeine De ce fait il est possible de séparer des bactéries à Gram positif et à Gram négatif présentant la même activité enzymatique.

Exemple 6 Utilisation d'un substrat à base d'Alizarine, l'Alizarine-beta-N-acétylglucosaminide (Aliz-β-NAG) avec un substrat à base d'Indoxyl le 5-Bromo-4-chloro-3-indolyl-beta-N-acetylglucosaminide (X-β-NAG) pour discriminer des bactéries et des levures possédant une activité hexosaminidase Un volume de 200 ml d'agar Columbia fondu à 50°C a été ajouté à différentes compositions en substrats résumées dans le tableau 9.

**Tableau 9**

| | Milieu 1 | Milieu 2 | Milieu 3 |
|---|---|---|---|
| Aliz-β-NAG | 50 mg/l | | 50 mg/l |
| X-β-NAG | - | 100 mg/l | 100 mg/l |

Les milieux ainsi constitués ont été répartis dans des boîtes de Petn. Différentes souches de microorganismes ont été ensemencées, à l'öese calibrée de 10 µl, sur chaque milieu, à partir de suspensions calibrées de 0,5 McFarland. Toutes les cultures ont été incubées 24 h à 37°C

Les résultats de croissance et de coloration obtenus à 24 h d'incubation sont donnés dans le tableau 6, dans lequel Co signifie croissance, C signifie couleur, I signifie incolore, In signifie intensité de coloration, le sigle ++ signifie très bonne croissance, le sigle + signifie bonne croissance de la souche, le sigle +/- signifie croissance moyenne de la souche et le sigle - signifie absence de croissance de la souche. 1 correspond à une intensité de coloration faible, 2 moyenne, 3 forte et 4 très forte.

**Tableau 10**

| | | Milieu 1 | | | Milieu 2 | | | Milieu 3 | | |
|---|---|---|---|---|---|---|---|---|---|---|
| | | C | In | Co | C | In | Co | C | In | Co |
| 1 | *Candida albicans* | violet | 2 | + | vert | 2 | + | bleu | 3 | + |
| 2 | *C. albicans* | Violet | 2 | + | vert | 2 | + | bleu | 3 | + |
| 3 | *E. faecalis* | violet | 3 | + | Vert | 1 | + | violet | 3 | + |
| 4 | *Enterobacter sakasakii* | violet | 3 | + | vert | 2 | + | Violet | 3 | + |
| 5 | *E. coli* | I | | + | I | | + | I | | + |

Les résultats dans le tableau 10 montrent que les bactéries hexosaminidase positive hydrolysent préférentiellement le substrat à base d'Alizarine alors que les levures hexosaminidase positive hydrolysent préférentiellement les substrats à base d'Indoxyl. De ce fait il est possible de séparer des bactéries de levures présentant la même activité enzymatique, en utilisant deux substrats pour cette même activité dont l'affinité de l'enzyme de chaque groupe est différente.

## Revendications

1. Procédé pour l'identification d'au moins deux groupes de microorganismes exprimant une même activité enzymatique comprenant les étapes suivantes :
a) l'incubation desdits groupes de microorganismes dans un milieu réactionnel comprenant un premier substrat enzymatique et un deuxième substrat enzymatique, lesdits premier et deuxième substrats enzymatiques étant métabolisés par une même activité enzymatique
b) l'identification desdits groupes de microorganismes

2. Procédé selon la revendication 1 selon lequel ladite même activité enzymatique est choisie parmi les activités enzymatiques suivantes : osidase, estérase, peptidase.

3. Procédé selon l'une des revendications 1 ou 2 selon lequel le milieu réactionnel comprend au moins un autre substrat métabolisé par au moins une autre activité enzymatique, ladite autre activité enzymatique étant préférentiellement choisie parmi une activité β-D-glucuronidase, β-glucosidase, tryptophanase, désaminase

## Patentansprüche

1. Ein Verfahren zur Identifizierung von mindestens zwei Gruppen von Mikroorganismen, die eine gleiche Enzymaktivität exprimieren, das die folgenden Schritte umfaßt:
a) die Inkubation von gesagten Gruppen von Mikroorganismen in einem Reaktionsmedium, das ein erstes Enzymsubstrat und ein zweites Enzymsubstrat beinhaltet, wobei das erste und das zweite Enzymsubstrat durch eine gleiche Enzymaktivität metabolisiert werden,
b) die Identifizierung der Gruppen von Mikroorganismen.

2. Verfahren gemäß Anspruch 1, wobei diese gleiche Enzymaktivität aus den folgenden Enzymaktivitäten ausgewählt wird : Osidase, Esterase, Peptidase.

3. Verfahren gemäß einem der Ansprüche 1 oder 2, wobei das Reaktionsmedium mindestens ein anderes Substrat beinhaltet, das durch mindestens eine andere Enzymaktivität metabolisiert wird, wobei die andere Enzymaktivität vorzugsweise aus einer ß-D-Glucuronidase-, ß-Glucosidase-, Tryptophanase-, Desaminase-Aktivität ausgewählt wird.

## Claims

1. A method for identifying at least two groups of microorganisms expressing the same enzymatic activity, comprising the following steps :
a) incubating said groups of microorganisms in a reaction medium comprising a first enzyme substrate and a second enzyme substrate, said first and second enzyme substrates being metabolized by the same enzymatic activity ;
b) identifying said groups of microorganisms.

2. The method as claimed in claim 1, wherein said same enzymatic activity is chosen from the following enzymatic activities : glycosidase, esterase and peptidase.

3. The method as claimed in claim 1 or 2, wherein the reaction medium also comprises at least one other substrate metabolized by at least one other enzymatic activity , said other enzymatic activity being preferably chosen from a beta-D-glucuronidase activity, a beta-glucosidase activity, a tryptophanase activity and a deaminase activity.
